# EUROPEAN PATENT SPECIFICATION

(11) **EP 2 529 715 B1**
(45) Date of publication and mention of the grant of the patent: **29.03.2017**
(21) Application number: 10844758.2
(22) Date of filing: 28.12.2010
(51) Int. Cl.: A61F 13/15, A61F 13/49, A61F 13/496

(54) **MANUFACTURING METHOD FOR ABSORBENT ARTICLE**
HERSTELLUNGSVERFAHREN FÜR SAUGFÄHIGEN ARTIKEL
PROCÉDÉ DE FABRICATION D'UN ARTICLE ABSORBANT

(30) Priority: 29.01.2010 JP 2010019808
(43) Date of publication of application: 05.12.2012
(73) Proprietor: Unicharm Corporation, Ehime 799-0111 (JP)
(72) Inventor: GOUDA, Hidefumi, Kanonji-shi Kagawa 769-1602 (JP); MUKAI, Hirotomo, Kanonji-shi Kagawa 769-1602 (JP)
(74) Representative: Peter, Julian
(86) International application number: PCT/JP2010/073908
(87) International publication number: WO 2011/093004

(56) References cited:
- JP-A- 1 014 303
- JP-A- 4 317 649
- JP-A- 7 136 210
- JP-A- 11 107 007
- JP-A- 63 099 302
- JP-A- 2003 339 768
- JP-A- 2008 228 760
- US-A1- 2006 185 135
- US-A1- 2008 105 384

## Description

### Technical Field

The present invention relates to a manufacturing method of an absorbent article.

### Background Art

A method is known in the prior art of manufacturing an underpants-type absorbent article, which is provided with an outer body provided with a front piece and a back piece mutually joined at side areas, the outer body having elastic members provided discontinuously along portions of the lower edges of the front piece and back piece that define the leg holes, and an inner body containing an absorptive body and which is anchored to the outer body so as to be positioned in a crotch area, including:
respectively transporting continuous sheets that respectively form the front piece and the back piece in the form of sheet webs in a machine direction, supplying a continuous elastic member in the form of a continuous elastic body to the sheet webs while oscillating in a cross-machine direction perpendicular to the machine direction so that portions of the continuous elastic body are alternately positioned on the sheet webs and outside the sheet webs, attaching the portions of the continuous elastic body positioned on the sheet webs to the sheet webs, affixing bands along the edges of the sheet webs to hold the continuous elastic body between the bands and the sheet webs, and cutting and removing the continuous elastic body together with the bands and the sheet webs (see PLT 1). In this manufacturing method, elastic members are not provided along portions of the lower edges of the front piece and back piece around the inner body positioned, and are therefore provided discontinuously.

### Citation List

### Patent Literature

PLT 1: International Publication No. WO 01/72237

Further prior art in this technical field is disclosed in documents US 2006/185135 A1 and US 2008/105384 A1.

### Summary of Invention

### Technical Problem

However, in the manufacturing method of PLT 1, since the bands and sheet webs are cut and removed, material that is not used in the finished product is required, thereby resulting in the risk of increasing material costs. Since the bands and sheet webs are cut and removed continuously along the edges of the sheet webs, the increase in cost in this case cannot be ignored.

In addition, since a device for forming and affixing the bands as well as space for installing that device are required, there is also the risk of an increase in costs required for production equipment

### Solution to Problem

According to the present invention, a manufacturing method of an underparits-type absorbent article according to claim 1 is provided.

### Advantageous Effects of Invention

An underpants-type absorbent article having elastic members around the leg holes can be manufactured inexpensively

### Brief Description of the Drawings

FIG. 1 is a perspective view of a diaper of a first embodiment according to the present invention.
FIG. 2 is a plan view of a diaper that has been opened up by unfastening joining portions positioned at side areas.
FIG. 3 is a longitudinal cross-sectional view taken along line III-III of FIG. 2.
FIG. 4 is a lateral cross-sectional view taken along line IV-IV of FIG. 2.
FIG. 5 is a general schematic diagram for explaining a method of manufacturing diapers.
FIG. 6 is partial schematic diagram for explaining a method of manufacturing diapers.
FIGS. 7(A) and 7(B) are drawings showing rollers R1 and R2.
FIG. 8 is a partial schematic diagram for explaining a method of manufacturing diapers.
FIGS. 9(A), 9(B), 9(C) and 9(D) are drawings of a top sheet web as viewed from arrows A, B, C and D of FIG. 6.
FIG. 10 is a drawing showing an adhesive application pattern.
FIGS. 11 (A) and 11(B) are drawings of other examples of adhesive application patterns.
FIG. 12 is a drawing for explaining a second embodiment according to the present invention.
FIGS. 13 (A) and 13(B) are drawings of a top sheet web as viewed from arrows A and B of FIG. 12.
FIG. 14 is a drawing for explaining a third embodiment according to the present invention.
FIG. 15 is an exploded plan view for explaining another example of an end portion.

### Description of Embodiments

The following provides an explanation of the present invention in the case of applying to a disposable underpants-type diaper. Note that the present invention can also be applied to other underpants-type absorbent articles worn in the manner of an undergarment.

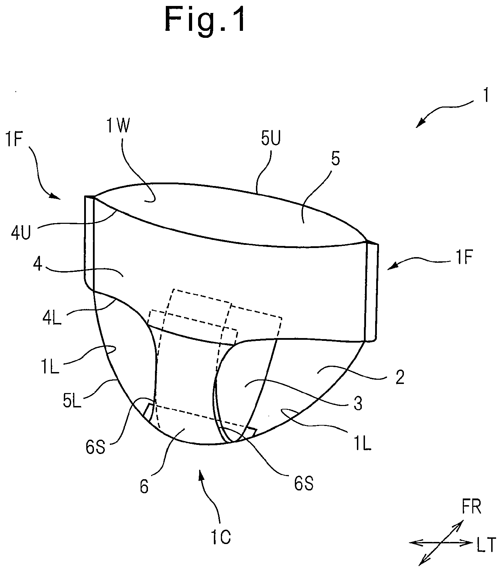

With reference to FIG. 1 showing a first embodiment according to the present disclosure, a diaper 1 is provided with an outer body 2 and an inner body 3.

The outer body 2 is provided with a mutually separate front piece 4, back piece 5 and connecting sheet 6. In addition to being mutually joined at side areas 1F, the front piece 4 and the back piece 5 are mutually connected by the connecting sheet 6 extending in the front-to-rear direction FR in a crotch area 1C. Here, when worn, the front piece 4 is positioned on the abdominal side of the wearer, and the back piece 5 is positioned on the back side of the wearer. In addition, the diaper 1 is provided with a waist opening or waist hole 1W defined by an upper edge 4U of the front piece 4 and an upper edge 5U of the back piece 5, and a pair of leg openings or leg holes 1L. In this case, each leg hole 1L is defined by an edge 1LE, and this edge 1LE is formed by a lower edge 4L of the front piece 4, a lower edge 5L of the back piece 5, and both edges 6S in the transverse direction LT of the connecting sheet 6. On the other hand, the inner body 3 contains an absorptive body, and is overlapped and anchored to the connecting sheet 6 on the inside of the outer body 2.

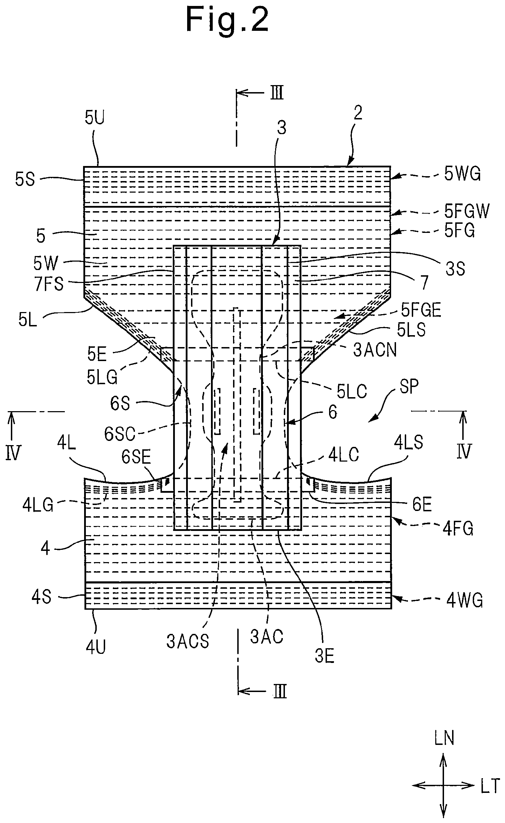

With reference to FIG. 2 showing the diaper 1 when opened up, the front piece 4 has a rectangular shape. The upper edge 4U of the front piece 4 extends linearly in the transverse direction LT. On the other hand, the lower edge 4L of the front piece 4 is provided with a central portion 4LC positioned substantially in the center in the transverse direction LT and side portions 4LS on both sides in the transverse direction LT of the central portion 4LC, the central portion 4LC extends substantially in transverse direction LT, and the side portions 4LS are slightly curved towards the upper edge 4U. In addition, both side edges 4S in the transverse direction of the front piece 4 extend substantially in a longitudinal direction LN. Here, the longitudinal direction LN is perpendicular to the transverse direction LT.

In addition, the back piece 5 has a hexagonal shape protruding towards the front piece 4. Namely, the back piece 5 is provided with a rectangular waist portion 5W and a trapezoidal extending portion 5E that extends from the waist portion 5W towards the front piece 4. The upper edge of the back piece 5, namely the upper edge 5U of the waist portion 5W, extends substantially in the transverse direction LT. On the other hand, the lower edge of the back piece 5, namely the lower edge 5L of the extending portion 5E, is provided with a central portion 5LC positioned substantially in the center in the transverse direction LT and side portions 5LS on both sides in the transverse direction LT of the central portion 5LC, the central portion 5LC extends substantially in the transverse direction LT, and the side portions 5LS are slightly curved towards the upper edge 5U while extending at an angle with respect to the transverse direction LT. In addition, both side edges 5S in the transverse direction of the back piece 5 extend substantially in the longitudinal direction LN.

When opened up as shown in FIG. 2, the front piece 4 and the back piece 5 are arranged while separated by a spacing area SP in the longitudinal direction LN perpendicular to the transverse direction LT. The connecting sheet 6 extends in the longitudinal direction LN by straddling this spacing area SP substantially in the center in the transverse direction LT of the front piece 4 and the back piece 5, and is respectively anchored to the front piece 4 around the lower edge 4L and the back piece 5 around the lower edge 5L, namely the extending portion 5E.

The connecting sheet 6 has a shape in which constrictions are provided in both sides of a rectangular shape substantially in the center in the longitudinal direction LN. In this case, the length and/or width of the connecting sheet 6 in the transverse direction LT is shorter than the width of the front piece 4 and the back piece 5 in the transverse direction LT, and the length of the connecting sheet 6 in the longitudinal direction LN is also shorter than the length of the inner body 3 in the longitudinal direction LN. In addition, the side edges 6S of the connecting sheet 6 are provided with a central portion 6SC substantially in the center in the longitudinal direction LN and end portions 6SE positioned on both sides in the longitudinal direction of the central portion 6SC, the central portion 6SC is curved inward, and the end portions 6SE extend substantially in the longitudinal direction LN. In other words, the end portions 6SE do not coincide with the edges of the outer body 2 that define the leg holes 1L, namely the lower edge 4L of the front piece 4 and the lower edge 5L of the back piece 5, but rather are positioned farther to the inside in the transverse direction LT than the lower edges 4L and 5L. Note that the end portions 6SE correspond to both edges in the transverse direction. LT of the portions of the connecting sheet 6 that overlap with the front piece 4 and the back piece 5. In addition, both end edges 6E in the longitudinal direction LN of the connecting sheet 6 extend substantially in the transverse direction LT.

On the other hand, the inner body 3 has a rectangular shape that extends in the longitudinal direction LN. Both side edges 3S in the transverse direction LT of the inner body 3 extend substantially in the longitudinal direction LN, while both end edges 3E in the longitudinal direction LN extend substantially in the transverse direction.

In addition, a pair of leakage preventing members 7 is provided on both sides in the transverse direction LT of the inner body 3 along both side edges 3S of the inner body 3. The free edges and/or outer edges 7SO in the transverse direction LT of the leakage preventing members 7 extend in the longitudinal direction LN.

As was previously described, the inner body 3 is overlapped and anchored on the connecting sheet 6 so as to be positioned in the crotch area 1C. In this case, the inner body 3 extends in the longitudinal direction LN beyond the connecting sheet 6 and is also anchored to the front piece 4 and the back piece 5.

In addition, in this case, at least a portion of the central portion 6SC of both side edges 6S of the connecting sheet 6 are positioned farther to the inside in the transverse direction LT than the outer edges 7SO of the leakage preventing members 7, and are also positioned farther to the inside in the transverse direction LT than both side edges 3S of the inner body 3. In contrast, the end portions 6SE of both side edges 6S are positioned farther to the outside in the transverse direction LT than both side edges 3S of the inner body 3, and are also positioned farther to the outside in the transverse direction LT than the outer edges 7SO of the leakage preventing members 7.

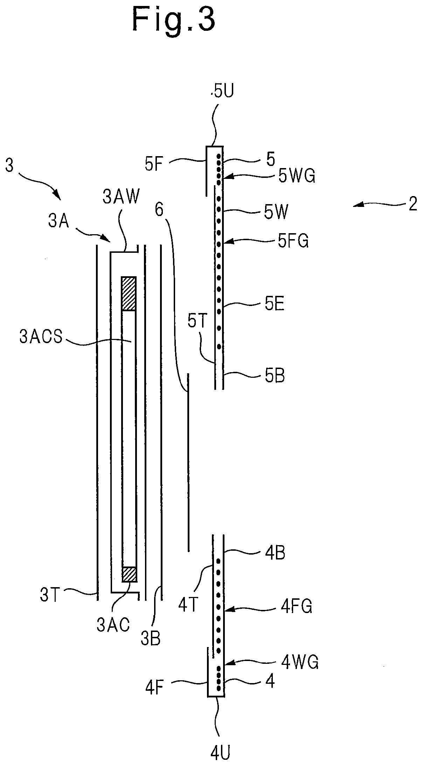

As shown in FIG. 3, the front piece 4 is provided with two sheets, namely, a top sheet 4T that faces the wearer when worn and a back sheet 4B that faces to the outside when worn, and the top sheet 4T and the back sheet 4B are mutually overlapped. Similarly, the back sheet 5 is also provided with two sheets, namely, a top sheet 5T that faces the wearer when worn and a back sheet 5B that faces to the outside when worn, and the top sheet 5T and the back sheet 5B are mutually overlapped.

On the other hand, the connecting sheet 6 is formed from a single sheet. The sheet in this case may be liquid permeable or liquid impermeable.

This being the case, the connecting sheet 6 is softer than the front piece 4 and the back piece 5. Note that the quantity, material or basis weight and the like of the sheet that forms the connecting sheet 6 can be selected so as to be softer than the front piece 4 and the back piece 5. The degree of softness of the sheet material can be measured by, for example, a cantilever method.

In addition, in the example shown in FIG. 2, the connecting sheet 6 is attached to the inside and/or wearer side of the front piece 4 and the back piece 5, namely to the top sheets 4T and 5T. However, the connecting sheet 6 may also be attached to the outside of the front piece 4 and the back piece 5, namely to the back sheets 4B and 5B. Alternatively, the areas around both ends in the longitudinal direction LN of the connecting sheet 6 may be made to be sandwiched between the top sheets 4T and 5T and between the back sheets 4B and 5B. Whereupon, both areas around the ends of the connecting sheet in the longitudinal direction LN are not exposed on the sides of the top sheets 4T and 5T or the back sheets 4B and 5B, thereby resulting in better touch to the skin. In addition, the connecting sheet 6 is more resistant to separation from the front piece 4 and the back piece 5.

Here, the top sheets 4T and 5T and back sheets 4B and 5B of the front piece 4 and the back piece 5 as well as the connecting sheet 6 are each a non-woven fabric formed from synthetic fibers such as polyolefin-based fibers in the manner of polypropylene (PP) or polyethylene (PE) or polyethylene terephthalate (PET)-based fibers, are formed from a non-woven fabric manufactured by spun bonding or air-through bonding, and each has a basis weight of, for example, 13 g/m² to 30 g/m². In the first embodiment according to the present disclosure, the top sheets 4T and 5T are respectively formed from an SMS non-woven fabric having a basis weight of 15 g/m² formed from PSS, the back sheets 4B and 5B are respectively formed from a spun-bonded non-woven fabric having a basis weight of 17 g/m² formed from PP, and the connecting sheet 6 is formed from an SMS non-woven fabric having a basis weight of 15 g/m² formed from PP.

In addition, an elastic member 4WG is provided in the front piece 4 around the upper edge 4U, and an elastic member 4FG is provided in the front piece 4 between the elastic member 4WG and the lower edge 4L. Moreover, elastic members 4LG are provided along the side portions 4LS of the lower edge 4L. As shown in FIG. 3, a folded portion 4F is provided around the upper edge 4U in which the back sheet 4B is folded to the side of the top sheet 4T, and the elastic member 4WG is anchored between the back sheets 4B at the folding portion 4F. On the other hand, the elastic members 4FG and 4LG are anchored between the top sheet 4T and the back sheet 4B.

Similarly, an elastic member 5WG is provided in the back piece 5 around the upper edge 5U, and an elastic member 5FG is provided in the back piece 5 between the elastic member 5WG and the lower edge 5L. Moreover, elastic members 5LG are provided along the side portions 5LS of the lower edge 5L. A folded portion 5F is provided around the upper edge 5U in which the back sheet 5B is folded to the side of the top sheet 5T, and the elastic member 5WG is anchored between the back sheets 5B at the folding portion 5F. On the other hand, the elastic members 5FG and 5LG are anchored between the top sheet 5T and the back sheet 5B, These elastic members 4WG, 4FG, 4LG, 5WG, 5FG and 5LG are in the form of threads, for example, and are attached to the front piece 4 and the back piece 5 while stretched in the transverse direction LT.

In this case, the elastic member 5FG includes an elastic member 5FGW provided in the waist portion 5W of the back piece 5 and an elastic member 5FGE provided in the extending portion 5E. As can be understood from FIG. 2, the elastic member 5FGE is provided overlapping the inner body 3 and the leakage preventing member 7. This elastic member 5FGE provides elastic action between the side areas 1F and crotch area 1C of the diaper 1 when worn. Similarly, the elastic members 4LG and 5LG also provide elastic action between the side areas 1F and crotch area 1C of the diaper 1 when worn.

Note that the elastic members 4LG and 5LG are not provided around the central portions 4LC and LC of the respectively corresponding lower edges 4L and 5L, and are thus provided discontinuously. Consequently, the elastic members 4LG and 5LG do not extend across the inner body 3 and/or connecting sheet 6 in the transverse direction LT. However, as shown in FIG. 2, the elastic members 4LG and 5LG slightly overlap the connecting sheet 6 and the inner body 3.

Note that the upper edges of the top sheets 4T and 5T are arranged roughly along the lower edges of the elastic members 4WG and 5WG, while the lower edges of the top sheets 4T and 5T are arranged roughly along the lower edges of the back sheets 4B and 5B.

Here, the elastic members 4WG, 4FG, 4LG, 5WG, 5FG and 5LG are formed from elastic fibers in the manner of natural rubber, synthetic rubber or spandex fibers, and have a draw ratio of, for example, 1.3 to 3.5. In the case the elastic members 4WG, 4FG, 5WG and 5FG are formed from spandex, the thickness of the elastic members 4WG, 4FG, 5WG and 5FG is, for example, 300 dtex to 1200 dtex. In the first embodiment according to the present disclosure, the elastic members 4WG and 5WG are respectively formed from, spandex having a thickness of 940 dtex and draw ratio of 3.0, the elastic members 4FG and 5FG are formed from spandex having a thickness of 780 dtex and draw ratio of 2.5, the elastic members 4LG are formed from spandex having an average draw ratio of 2.5, and the elastic members 5LG are formed from spandex having an average draw ratio of 2.2. In addition, the elastic member 4WG and the elastic member 5WG respectively contain 5 spandex fibers, the elastic member 4FG contains 10 spandex fibers, the elastic member 5FGW of the waist portion 5W contains 11 spandex fibers, and the elastic member 5FGE of the extending portion 5E contains 2 spandex fibers. Note that the elastic members 4WG, 4FG, 4LG, 5WG, 5FG and 5LG may also be formed from elastic sheets.

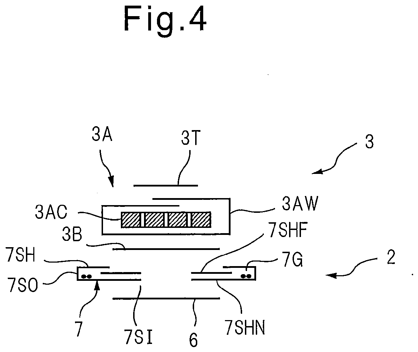

As is shown in FIGS. 3 and 4, the inner body 3 is provided with a liquid permeable top sheet 3T, a liquid impermeable back sheet 3B and an absorptive body 3A arranged between the top sheet 3T and the back sheet 3B. In addition, the absorptive body 3A is provided with an absorptive body core 3AC and a wrapping sheet 3AW that envelops the absorptive body core 3AC.

Widened portions 3ACW are formed on both ends in the lengthwise direction and/or longitudinal direction LN of the absorptive body core 3AC and at an intermediate portion between both ends, and these widened portions 3ACW are mutually connected by narrowed portions 3ACN.

In addition, a plurality of slits 3ACS are provided in the absorptive body core 3AC that extend in the lengthwise direction and/or longitudinal direction LN. These slits 3ACS include a central slit positioned substantially in the center in the transverse direction LT, and side slits positioned on both sides of the central slit. In the first embodiment according to the present disclosure, the length of the central slit is 320 mm and the width is 12 mm, while the length of the side slits is 80 mm and the width is 10 mm. This being the case, the absorptive body core 3AC easily bends along the slits 3ACS, thereby facilitating adherence of the absorptive body 3A to the wearer.

The top sheet 3T is formed from a hydrophilic non-woven fabric formed from polyolefin-based fibers or PET fibers and the like that is manufactured by a manufacturing method such as spun bonding or air-through bonding. The back sheet 3B is formed from a water-resistant and moisture-permeable film formed from PE and the like. The absorptive body core 3AC is formed from pulp, superabsorbent polymer (SAP) or mixture thereof. The wrapping sheet 3AW is formed from a hydrophilic non-woven fabric formed from polyolefin-based fibers or PET fibers and the like that is manufactured by a manufacturing method such as spun bonding or air-through bonding. In the first embodiment according to the present disclosure, the top sheet 3T is formed from a non-woven fabric manufactured by air-through bonding and having a basis weight of 25 g/m², the back sheet 3B is formed from a moisture-permeable PET film having a basis weight of 22. g/m², the absorptive body core 3AC is formed from a single mixed layer of pulp having a basis weight of 250 g/m² and SAP having a basis weight af 200 g/m², and the wrapping sheet 3AW is formed from an SMS non-woven fabric having a basis weight of 13 g/m².

As is shown in FIG. 4, each of the leakage preventing members 7 has anchored edges and/or inner edges 7SI anchored to the outer body 2 or the inner body 3, and free edges and/or outer edges 7SO not anchored to the outer body 3 or the inner body 2. In addition, each of the leakage preventing members 7 includes a liquid impermeable sheet 7SH and elastic members 7G, and the liquid impermeable sheet 7SH includes a leakage preventing non-woven fabric 7SHN and a leakage preventing film 7SHF. In the outer edges 7SO of each of the leakage preventing members 7, the leakage preventing non-woven fabric 7SHN is folded and overlaps the outer edges of the leakage preventing film 7SHF, and the elastic members 7G are anchored between the folded leakage preventing non-woven fabric 7SHN. Note that the outer edges of the leakage preventing film 7SHF do not reach to the outer edges 7SO of the leakage preventing members 7. On the other hand, in the inner edges 7SI of each of the leakage preventing members 7, the edges of the leakage preventing non-woven fabric 7SHN and the leakage preventing film 7SHF are substantially mutually arranged in rows.

When worn, each of the leakage preventing members 7 rises up towards the wearer to act as leakage preventing barriers.

The leakage preventing non-woven fabric 7SHN is formed from polyolefin-based fibers or PET fibers and the like, and is manufactured by a method such as spun bonding. The leakage preventing film 7SHF is formed from PE or PET and the like. In the first embodiment according to the present disclosure, the leakage preventing non-woven fabric 7SHN is formed from hydrophobic SMS non-woven fabric having a basis weight of 15 g/m², and the leakage preventing film 7SHF is formed from a moisture-permeable PE film having a basis weight of 18 g/m². The elastic members 7G are formed from elastic fibers in the manner of natural rubber, synthetic rubber or spandex fibers. In the first embodiment according to the present disclosure, the elastic members 7G are formed from two spandex fibers having a thickness of 620 dtex and draw ratio of 2.2.

Connection and/or anchoring of these members are carried out by, for example, heat sealing, sonic sealing or an adhesive and the like. A hot melt adhesive (HMA) containing, for example, styrene-isoprene-styrene (SIS), styrene-butadienestyrene (SBS) or styrene-ethylene-butylene-styrene (SEBS) adhesive can be used for the adhesive.

More specifically, the top sheets 4T and 5T and back sheets 4B and 5B of then front piece 4 and the back piece 5 are mutually connected to the elastic members 4WG, 4FG, 5WG and 5FG with a preliminarily applied HMA. In addition, at those locations where the elastic members 4WG, 5FG, 5WG and 5FG are sparse, namely where the interval between elastic members is, for example, 10 mm or more, HMA is applied to the top sheets 4T and 5T and the back sheets 4B and 5B by a coating method in the manner of spiral coating or controlled seam coating. Moreover, HMA is also applied to the top sheets 4T and 5T and the back sheets 4B and 5B around the lower edges 4L and 5L in order to prevent separation.

On the other hand, the elastic members 4LG and 5LG are anchored to the top sheets 4T and 5T by HMA preliminarily applied to the top sheets 4T and 5T by a coating method such as spiral coating. In this case, the HMA is applied so as to follow the loci of the elastic members 4LG and 5LG.

In addition, the connecting sheet 6 is connected to the front piece 4 and the back piece 5 by a method such as sonic sealing or adhesive. In the first embodiment according to the present disclosure, HMA is preliminarily applied to the back of the connecting sheet 6 by a coating method such as slot coating.

On the other hand, in the inner body 3, the top surface and bottom surface of the absorptive body core 3AC are connected to the core wrapping sheet 3AW by an adhesive such as HMA. In this case, the adhesive is applied by a coating method such as spiral coating, slot coating, controlled seam coating, bead coating or curtain coating so as to have a basis weight of 1.5 g/m² to 10 g/m². In the first embodiment according to the present disclosure, HMA is applied by a spiral coating method so as to have a basis weight of 5 g/m².

In addition, HMA is applied to the back sheet 3B of the inner body 3 or side of the leakage preventing members 7 facing the outer body 2 by a controlled seam coating method, and as a result thereof, the inner body 3 and the leakage preventing members 7 are anchored to the front piece 4, the back piece 5 and the connecting sheet 6.

In the leakage preventing members 7, HMA applied to the elastic members 7G by a slit nozzle method, and the elastic members 7G are anchored to the leakage preventing non-woven fabric 7SHN by this HMA. In addition, the leakage preventing non-woven fabric 7SHN and the leakage preventing film 7SHF are mutually joined by applying HMA to the leakage preventing non-woven fabric 7SHN by spiral coating

Note that, in the side areas 1F of the diaper 1, the front piece 4 around both side edges 4S and the back piece 5 around both side edges 5S are mutually joined by a method such as heat sealing or ultrasonic sealing. Note that the front piece 4 and the back piece 5 may be refastenably joined, and in this case, mechanical fasteners containing hooks and loops can be used.

The edges 1LE that define the legs holes 1L are formed by the side portions 4LS of the lower edge 4L of the front piece 4, the side portions 5LS of the lower edge 5L of the back piece 5, and both edges 6S of the connecting sheet 6. In this case, the edges 1LE are formed by a single cutting action and are smoothly curved and continuing.

In the first embodiment according to the present disclosure, at least a portion of both side edges 6S of the connecting sheet 6 are positioned farther to the inside in the transverse direction LT than both side edges 3 S of the inner body 3 in the spacing area SP between the front piece 4 and the back piece 5 as was previously described. Thus, the range of movement of the legs of a wearer can be increased while maintaining a large absorptive surface for the inner body 3. Namely, difficulty encountered by a wearer when moving the legs can be inhibited while maintaining favorable absorptive performance of the inner body 3.

At the same time, the end portions 6SE of the connecting sheet 6 are positioned farther to the outside in the transverse direction LT than both side edges 3S of the inner body 3 (FIG. 2). As a result, the front piece 4 and the back piece 5 can be reliably connected to the connecting sheet 6 while facilitating movement of the legs of a wearer.

Moreover, since the extending portion 5E having the elastic member 5FGE is provided in the back piece 5, the shape of the diaper I can be made to be that of an undergarment, and the entire gluteal region of the wearer is covered by the back piece 5. Thus, leakage can be suppressed and apprehension of the wearer concerning leakage can be reduced.

Moreover, since the inner body 3 is pulled up towards the side areas 1F (FIG. 1) by the elastic members 5FGE, 4LG and 5LG, twisting of the inner body 3 to the inside is suppressed, thereby maintaining absorptive properties of the inner body 3.

What is more, since the elastic members 4LG and 5LG are provided discontinuously, namely since the elastic members 4LG and 5LG extend by straddling the inner body 3 and/or the connecting sheet 6, the inner body 3 can be prevented from shrinking and decreasing in absorption area. In addition, compression of the groin region of a wearer can be prevented. Moreover, as a result of providing the elastic members 4LG and 5LG, the elastic member 5FGE can be omitted, thereby making it possible to suppress undesirable deformation of the inner body 3.

Moreover, since the edges 1LE that define the leg holes 1L are continuing by curving smoothly, they have a curved shape that follows the legs of a wearer, thereby enhancing adherence to the wearer. In this case, adherence is further enhanced by the elastic members 4LG and 5LG.

Moreover, the length of the connecting sheet 6 can be shorter than the length of the inner body 3. Thus, the amount of the connecting sheet 6 can be decreased, thereby making it possible to reduce costs.

Moreover, the connecting sheet 6 is softer than the front piece 4 and the back piece 5 as was previously described. As a result, in comparison with the case of having two sheets in the crotch area 1C as a result of the entire outer body 2 being provided with a top sheet and a back sheet, the connecting sheet and/or inner body 2 makes soft contact with the legs and/or groin region of a wearer and the legs of the wearer become easier to move. In addition, even if the outer body 2 rolls up and becomes wrinkled, the wearer is less likely to feel a sense of tightness. Moreover, since the connecting sheet 6 is formed from a single sheet, costs can be reduced in comparison with the entire outer body 2 formed from a top sheet and back sheet.

In addition, since the connecting sheet 6 can be formed from a material differing from that of the front piece 4 and the back piece 5, cost performance of the diaper 1 can be enhanced. Namely, if the connecting sheet 6 is formed from a moisture permeable or moisture absorptive material, breathability in the crotch area 1C can be improved. A non-woven fabric containing cellulose-based fibers such as rayon or pulp and polyester fibers can be used as a moisture absorptive material. More specifically, a non-woven fabric containing rayon, polyolefin-based fibers and polyester fibers (such as a non-woven fabric manufactured by spun lacing and having a basis weight of 26 g/m²) or a non-woven fabric containing pulp and polyester (such as a non-woven fabric manufactured by spun lacing and having a basis weight of 40 g/m²) is used.

In addition, twisting of the inner body 3 to the inside can be suppressed by providing the connecting sheet 6. Consequently, the leakage preventing members 7 reliably rise up towards the wearer. Namely, the connecting sheet 6 provides a starting point for the leakage preventing members 7 to rise up towards the wearer.

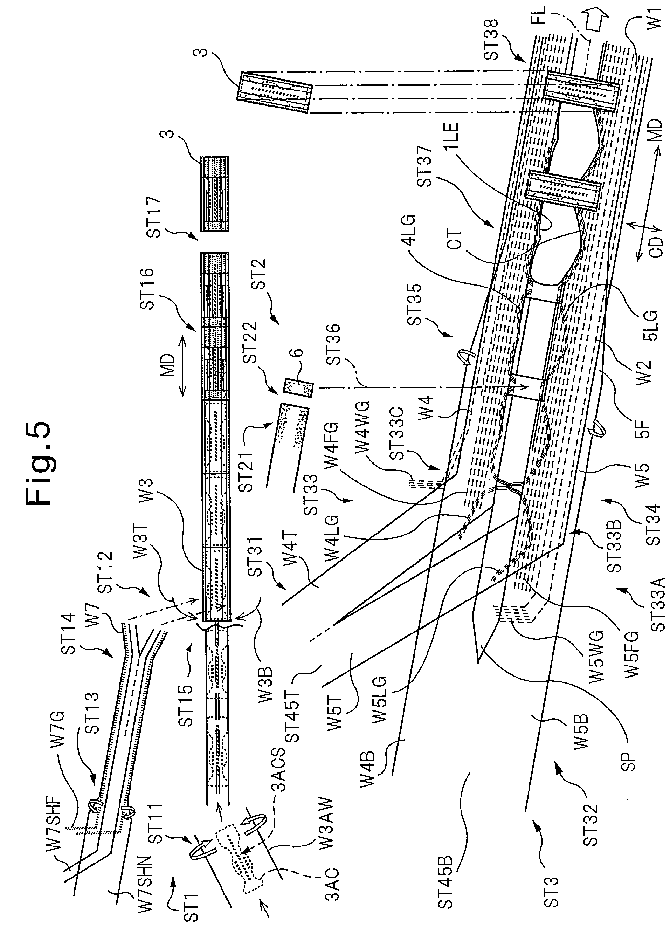

Next, an explanation is provided of the manufacturing method of the diaper 1 of the first embodiment according to the present disclosure with reference to FIG. 5.

With reference to FIG. 5, the manufacturing method of the first embodiment according to the present invention comprises a step ST1 for manufacturing the inner body 3 provided with the leakage preventing members 7, a step ST2 for manufacturing the connecting sheet 6, and a step ST3 for manufacturing a continuous body of the diaper 1 in the form of a diaper web W1.

In the step ST1, the absorptive body core 3AC in which the slits 3ACS have been preliminarily formed is first wrapped with a continuous body of the wrapping sheet 3AW in the form of a wrapping sheet web W3AW (ST11). Note that, in this case, the absorptive body core 3AC is transported so that the slits 3ACS are parallel to a machine direction MD.

Next, continuous bodies of the top sheet 3T and back sheet 3B in the form of top sheet web W3T and back sheet web W3B are respectively affixed to the upper surface and lower surface of the wrapping sheet web W3AW wrapped around the absorptive body core 3AC to form a continuous body of the inner body 3 in the form of an inner body web W3 (ST12).

On the other hand, a continuous body equivalent to two leakage preventing films 7SHF in the form of a leakage preventing film web W7SHF and a continuous body of the elastic members 7G in the form of continuous elastic bodies W7G are attached to a continuous body equivalent to two leakage preventing non-woven fabrics 7SHN in the form of a leakage preventing non-woven fabric web W7SHN (ST13). Next, the leakage preventing non-woven fabric web W7SHN is divided along the machine direction MD to form a continuous body of the leakage preventing members 7 in the form of leakage preventing member webs W7 (ST14).

Next, each leakage preventing member web W7 is affixed to the back of the inner body web W3, namely the back sheet web W3B (ST15).

Next, HMA is applied in a predetermined pattern to the back of the inner body web W3 and the leakage preventing member webs W7, namely the side opposing an outer body web W2 (ST16).

Next, the inner body web W3 and the leakage preventing member webs W7 are cut to the length of a single finished product to form the inner body 3 provided with the leakage preventing members 7 (ST17).

In step ST2, HMA is applied to the bottom of a continuous body of the connecting sheet 6 in the form of a connecting sheet web W6 (ST21).

Next, the connecting sheet web W6 is cut to the length of a single finished product to form the connecting sheet 6 (ST 22).

In step ST3, a top sheet web W45T is divided along the machine direction MD to form a continuous body of the top sheet 4T of the front piece 4 in the form a top sheet web W4T and a continuous body of the top sheet 5T of the back piece 5 in the form of a top sheet web W5T (ST31). Similarly, a back sheet web W45B is divided along the machine direction MD to form a continuous body of the back sheet 4B of the front piece 4 in the form of a back sheet web W4B and a continuous body of the back sheet 5 of the back piece 5 in the form of a back sheet web W5B (ST32). The top sheet web W4T and the back sheet web W5B are transported along the machine direction MD separated by the spacing area SP in a cross-machine direction CD substantially perpendicular to the machine direction MD

In addition, continuous bodies of the elastic members 4WG and 5WG in the form of continuous elastic bodies W4WG and W5WG as well as continuous bodies of the elastic members 4FG and 5FG in the form of continuous elastic bodies W4FG and W5FG are respectively attached to the back sheet webs W4B and W5B while being stretched in the machine direction MD (ST33).

In addition, continuous bodies of the elastic members 4LG and 5LG in the form of continuous elastic bodies W4LG and W5LG are respectively attached to the top sheet webs 4WT and W5T while being stretched (ST33A). In this case, although subsequently described in detail, portions of the continuous elastic bodies W4LG and W5LG are positioned on the top sheet webs W4T and W5T, while the remaining portions are positioned in the spacing area SP, namely outside the top sheet webs W4T and W5T.

Next, the top sheet webs W4T and W5T are respectively overlapped with the back sheet webs W4B and W5B, and as a result thereof, the continuous elastic bodies W4WG, W4FG, W4LG, W5SG, W5FG and W5LG are respectively sandwiched between the back sheet webs W4B and W5B and the top sheet webs W4T and W5T (ST33B). Next, portions P4 and P5 of the continuous elastic bodies W4LG and W5LG positioned in the spacing area SP are cut (ST33C).

Next, the back sheets webs W4B and W5B and the top sheet webs W4T and W5T are pressed to form continuous bodies of the front piece 4 and the back piece 5 in the form of a front piece web W4 and a back piece web W5 (ST34).

In addition, the outer edges of the back sheet webs W4B and W5B are folded to form folded portions 5F (ST35).

In addition, the connecting sheet 6 is attached to the front piece web W4 and the back piece web W5 at intervals in the machine direction MD so as to straddle the spacing area SP. As a result, a continuous body of the outer body 2 in the form of the outer body web W2 is formed (ST36).

Note that, in the case of making the areas around both ends of the connecting sheet 6 in the longitudinal direction LN to be sandwiched between the top sheets 4T and 5T and the back sheets 4B and 5B, the connecting sheet 6 is first attached to the top sheet webs W4T and W5T, after which the back sheet webs W4B and W5B are overlapped with the connecting sheet 6 and the top sheet webs W4T and W5T. Alternatively, the connecting sheet 6 is first attached to the back sheet webs W4B and W5B, after which the top sheet webs W4T and W5T are overlapped with the connecting sheet 6 and the back sheet webs W4B and W5B.

Next, a cutting action is carried out on the outer body web W2 at an interval in the machine direction MD to form the edges 1LE that define the leg holes 1L (ST37). In this case, the front piece web W4 and the back piece web W5 are cut along the elastic members 4LG and 5LG. In addition, the connecting sheet 6 is cut so that both side edges 6S of the connecting sheet 6 are curved inward, or so that at least a portion of both side edges 6S of the connecting sheet 6 in the spacing area SP are positioned farther to the inside in the transverse direction LT than both side edges 3 S of the inner body 3. Note that cutting action is carried out once along an annular cutting line CT, for example. The trimmed material is recovered in this case.

Next, the inner body 3 provided with the leakage preventing members 7 is attached to the front piece web W4, the back piece web W5 and the connecting sheet 6 to form the diaper web W1 (ST38). Note that guides are preferably provided immediately before the cutting action and action for attaching the inner body 3 so that the position of the outer body web W2 in the cross-machine direction CD is at the proper position.

Next, the diaper web W1 is folded along a folding line FL lying in the machine direction MD (not shown). In this case, the folding line FL may be in the center of the diaper web W1 in the cross-machine direction CD or may be shifted from the center.

Next, the front piece web W4 and the back piece web W5 are partially joined at intervals in the machine direction MD to form joined portions as a result thereof. Next, the diaper web W1 is cut at these joined portions in the cross-machine direction CD to form the diaper 1 (not shown).

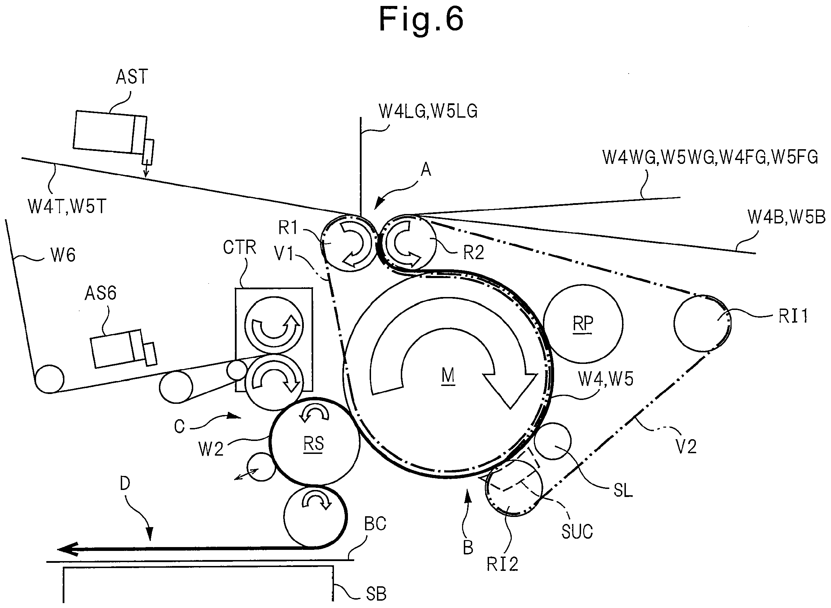

An explanation is provided of steps ST33, ST33A, ST33B, ST34, ST36 and the like with reference to FIG. 6, FIG. 7(A), FIG. 7(B), FIG. 8, FIG. 9(A), FIG. 9(B), FIG. 9(C) and FIG. 9(D). First, in providing a general explanation thereof, the top sheet webs W4T and W5T are respectively transported towards a roller R1, while the back sheet webs W4B and W5B are respectively transported towards a roller R2.

Prior to reaching the roller R1, the stretched continuous elastic bodies W4LG and W5LG are supplied and attached to the top sheet webs W4T and W5T while oscillating in the cross-machine direction CD. In this case, as was previously described, the continuous elastic bodies W4LG and W5LG are supplied so as to be alternately positioned within the space area SP on the top sheet webs W4T and W5T. Note that the portions P4 and P5 of the continuous elastic bodies W4LG and W5LG positioned in the spacing area SP are not required to be mutually overlapped. In addition, the portions P4 and P5 are made not to be positioned on the top sheet webs W5T and W4T.

In addition, prior to this, HMA is supplied from an HMA applicator AST to the top sheet webs W4T and W5T so as to match the arrangement pattern of the continuous elastic bodies W4LG and W5LG. As a result, those portions of the continuous elastic bodies W4LG and W5LG positioned on the top sheet webs W4T and W5T are attached to the top sheet webs W4T and W5T. On the other hand, prior to reaching the roller R2, stretched continuous elastic bodies W4WG, W5WG, W4FG and W5FG are supplied to the back sheet webs W4B and W5B.

Since the rollers R1 and R2 are mutually adjacent, the top sheet webs W4T and W5T and the back sheet webs W4B and W5B are overlapping on the rollers R1 and R2, respectively. At this time, the continuous elastic bodies W4WG, W5WG, W4FG and W5FG and the portions of the continuous elastic bodies W4LG and W5LG positioned on the top sheet webs W4T and W5T are sandwiched between the top sheet webs W4T and W5T and the back sheet webs W4B and W5B. Note that, as was previously described, HMA is preliminarily applied to the continuous elastic bodies W4WG, W5WG, W4FG and W5FG, and the continuous elastic bodies W4WG, W5WG, W4FG and W5FG are attached to the top sheet webs W4T and W5T and the back sheet webs W4B and W5B by this HMA.

At this time, the portions P4 and P5 of the continuous elastic bodies W4LG and W5LG positioned in the spacing area SP are held by an endless belt to be subsequently described, and are transported together with the top sheet webs W4T and W5T, the back sheet webs W4B and W5B and the like while still held.

The mutually overlapped top sheet webs W4T and W5T and back sheet webs W4B and W5B are then transported by a main drum M and pressed together by a press roller RP. As a result, the continuous elastic bodies W4WG, W5WG, W4FG and W5FG and portions of the continuous elastic bodies W4LG and W5LG are attached to the top sheet webs W4T and W5T and the back sheet webs W4B and W5B.

Next, the portions P4 and P5 of the continuous elastic bodies W4LG and W5LG are cut by a slicer SL while being held by the endless belt. The cut portions P4 and P5 are transported while held by the endless belt, and are then sectioned and removed by a suction pump SUC arranged adjacent to the main drum M. As a result, the front piece web W4 and the back piece web W5 are formed provided with the elastic members 4LG and 5LG.

Next, the front piece web W4 and the back piece web W5 reach a suction transfer roller RS and are transported while being suctioned and held thereby.

On the other hand, following application of HMA by the HMA applicator AS6, the connecting sheet web W6 is transported to a cutter CTR and cut to form the connecting sheet 6. This connecting sheet 6 is discontinuously attached to the front piece web W4 and the back piece web W5 on the suction transfer roller RS. The outer body web W2 is formed in this manner.

Next, the outer body web W2 is transported by a belt conveyor BC traveling over a suction box SB. This being the case, the outer body web W2 can be suppressed from shifting out of position and forming wrinkles. Note that the suction box SB is preferably formed so that suction action is applied to the front piece web W4 and the back piece web W5 without being applied to the spacing area SP.

Next, an explanation is further provided regarding holding and cutting the portions of the continuous elastic bodies W4LG and W5LG positioned in the spacing area SP.

As shown in FIG. 6, an endless belt V1 is wrapped around the roller R1 and the main drum M, and an endless belt V2 is wrapped around the roller R2, idle rollers R11 and R12 and the main drum M.

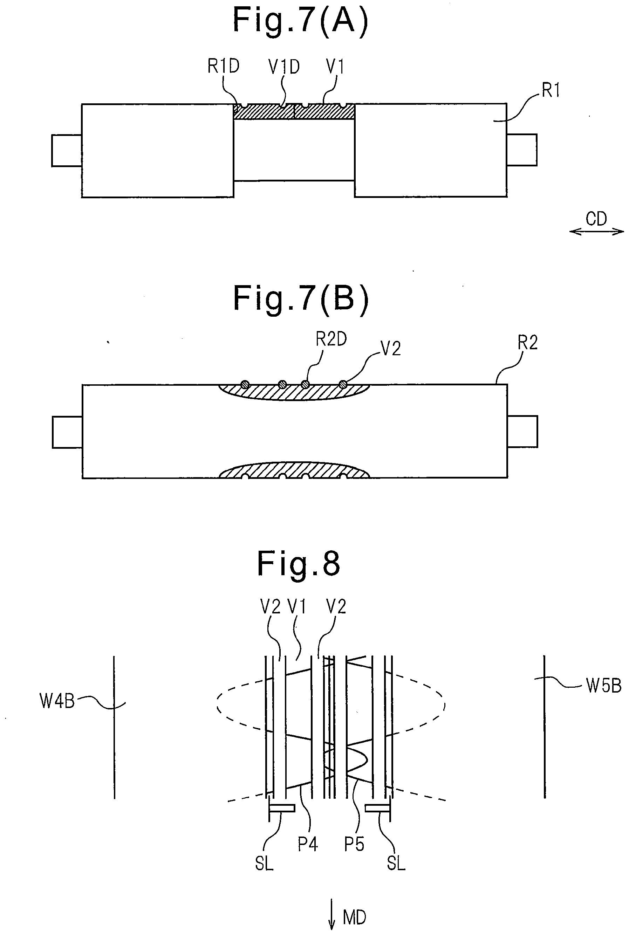

As shown in FIGS. 7(A) and 7(B), the endless belt V1 is formed from, for example two flat belts, while the endless belt V2 is formed from, for example, four wire belts having a circular cross-section. In this case, a single flat belt and two wire belts are used as a set. Note that the endless belt V1 may be formed from a wire belt, while the endless belt V2 may be formed from a flat belt.

As shown in FIG. 7(A), a concave groove RID that receives the endless belt V1 is formed in the center of the roller R1 in the axial direction. In addition, a similar concave groove is also formed in the center of the main drum M in the axial direction. This being the case, the endless belt V1 is suppressed from moving in the cross-machine direction CD. Note that concave portions and convex portions are respectively and alternately formed in the circumferential direction of the concave grooves of the roller R1 and the main drum M, and these concave portions and convex portions engages with concave portions and convex portions formed in the back of the endless belt V1. Moreover, a groove VID capable of receiving the corresponding endless belt V2 is formed in the endless belt V1. The shape of the surface of then groove VID is preferable complementary to the external shape of the endless belt V2.

On the other hand, as shown in FIG. 7(B), a concave groove R2D that receives the endless belt V2 is formed in the roller R2. Similar concave grooves are also formed in the idle rollers R11 and R12. This being the case, the endless belt V2 is suppressed from moving in the cross-machine direction CD.

In the first embodiment according to the present invention, the main drum M and the rollers R1 and R2 are driven in one direction, and as a result thereof, the top sheet webs W4T and W5T and the back sheet webs W4B and W5B are transported together with the endless belts V1 and V2.

The areas outside the top sheet webs W4T and W5T, namely the portions P4 and P5 of the continuous elastic bodies W4LG and W5LG positioned in the spacing area SP, are positioned on the endless belt V1 at the roller R1 Next, the endless belt V2 is overlapped with the endless belt V1, and as a result thereof, the portions P4 and P5 are held between these endless belts V1 and V2. In this case, the portions P4 and P5 can be held reliably since the endless belt V2 is received in the concave groove RID of the endless belt V1 together with the portions P4 and P5. Note that the positions of the endless belts V1 and V2 at the rollers R1 and R2 are preset so that the portions P4 and P5 are held between the endless belts V1 and V2.

The portions P4 and P5 are transported to the main drum M while held by the endless belts V1 and V2. Next, as shown in FIG. 8, the portions P4 and P5 are cut by the slicer SL between the endless belts V1 and V2 and the top sheet webs W4T and W5T and the like. In this manner, the endless belts V1 and V2 form holders that hold the portions P4 and P5. Note that, although a single endless belt V2 can also be provided for a single endless belt V1, a plurality of endless belts V2 are preferably provided.

A groove that receives the blade of the slicer SL can also be provided in the circumferential surface of the main drum M. This is because the diameters of the portions P4 and P5 become smaller as a result of being pulled by the slicer blade thereby making them easier to cut. In addition, a method can be used in which the cutter is pressed from one side, or a so-called sonic cutter can be used that cuts by vibrating a blade tip in a sharpened state.

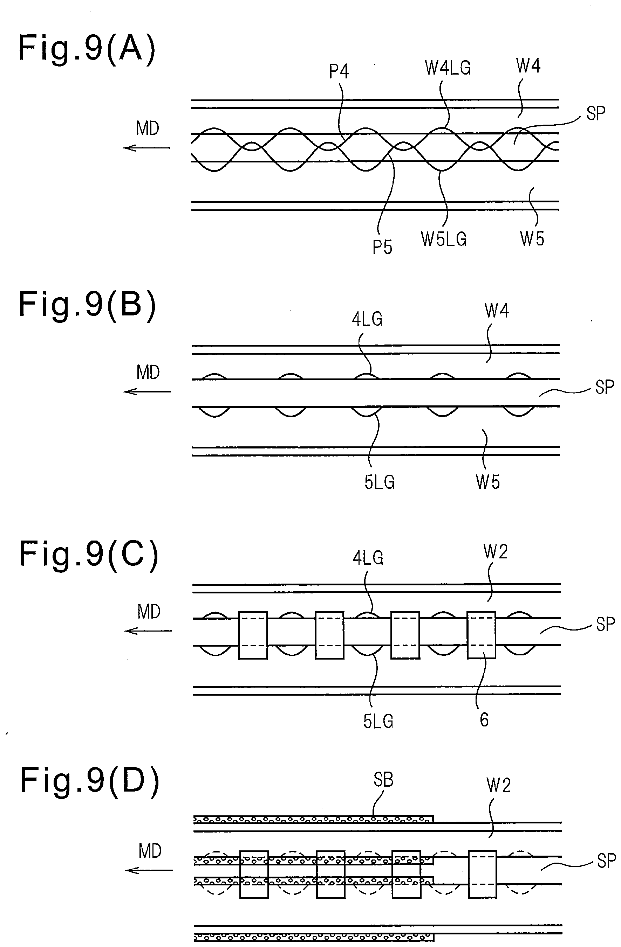

The top sheet webs W4T and W5T as viewed from arrows A, B, C and D of FIG. 6 are respectively shown in FIG. 9(A), FIG. 9(B), FIG. 9(C) and FIG. 9(D). Note that the continuous elastic bodies W4WG, W5WG, W4FG and W5FG are omitted from FIGS 9(A), 9(B), 9(C) and 9(D).

In this manner, in the first embodiment according to the present invention, the portions P4 and P5 are cut and removed without cutting and removing the top sheet webs W45 and W5T and the back sheet webs W4B and W5B. As a result, material is not wasted and materials costs can therefore be reduced considerably.

Note that, since the top sheet webs W4T and W5T and the back sheet webs W4B and W5B are not cut, the corresponding edges of the top sheet web W4T and the back sheet web W4B that face the spacing area SP and the corresponding edges of the top sheet web W5T and the back sheet web W5B that face the spacing area SP remain as when the top sheet web W4T and the back sheet web W4B were mutually overlapped, or in other words, are not always mutually aligned. In contrast, in the case the top sheet webs W4T and W5T and the back sheet webs W4B and W5B are cut along with the portions P4 and P5, the edges of the top sheet webs W4T and W5T and the edges of the back sheet webs W4B and W5B are respectively mutually aligned.

In addition, in this case, since the top sheet webs W4T and W5T and the back sheet webs W4B and W5B are not damaged, shifting of position by the continuous elastic bodies W4WG, W4FG, W4LG, W5WG, W5FG and W5LG positioned between the top sheet webs W4T and W5T and the back sheet webs W4B and W5B can be prevented. Moreover, wrinkling of the top sheet webs W4T and W5T and the back sheet webs W4B and W5B can be prevented, thereby enabling the manufacturing of a finished product that also has an attractive appearance.

Moreover, if the continuous elastic bodies W4LG and W5LG are supplied so that the draw ratios of the portions P4 and P5 are larger than those of other portions, the amounts of the portions P4 and P5 to be discarded can be reduced. Alternatively, the supply path of the continuous elastic bodies W4LG and W5LG may also be set so that the lengths of the portions P4 and P5 become shorter.

In addition, in the first embodiment according to the present invention, cutting action for forming the edges ILE that define the leg holes 1L is carried out after the connecting sheet 6 is connected to the front piece web W4 and the back piece web W5. As a result, cutting action can be carried out with the outer body web W2, namely the front piece web W4 and the back piece web W5 mutually connected to the connecting sheet 6, free of wrinkles as a result of respectively pulling to the outside in the cross-machine direction CD. Thus, the leg holes 1L can be formed in the proper shape. In addition, the outer body 3 can be attached to the inner body web 2 without wrinkling. Thus, the inner body 3 can be reliably attached to the inner body web W2.

Namely, there is the risk of wrinkling of the front piece web W4 and the back piece web W5 in the state in which they are not mutually connected by the connecting sheet 6, and if cutting action is carried out while in this state, there is the risk of the edges 1LE that define the leg holes 1L deviating from the proper shape. In addition, it is also difficult to reliably attach the inner body 3 to the outer body web W2 while in this state. On the other hand, when cutting action is carried out, the front piece web W4 and the back piece web W5 around the edges 1LE that define the leg holes 1L become discontinuous in the machine direction MD. Since these discontinuous portions are not held in the machine direction MD, there is the risk of these portions being rolled up and flapping about, thereby causing wrinkling or flapping of the front piece web W4 and the back piece web W5. Moreover, when such discontinuous portions are formed, the continuous elastic bodies W4LG and W5LG, for example, of the front piece web W4 and the back piece web W5 contract in the crass-machine direction CD, again resulting in the risk of wrinkling or flapping of the front piece web W4 and the back piece web W5. When the connecting sheet 6 or the inner body 3 is attempted to be attached to the front piece web W4 and the back piece web W5 in which wrinkling or flapping has occurred, the connecting sheet 6 or the inner body 3 is not reliably connected to the front piece web W4 and the back piece web W5, thereby resulting in the risk of a decrease in productivity of the diaper 1.

In contrast, in the first embodiment of the present invention, after the front piece web W4 and the back piece web W5 are mutually connected by the connecting sheet 6, cutting action is carried out for forming the edges 1LE followed by attachment of the inner body 3. Thus, problems like those described above do not occur. Note that these problems are unique to methods of manufacturing the diaper 1 in which the front piece 4 and the back piece 5 are formed from separate sheets, and do not occur in methods of manufacturing diapers in which the front piece and the back piece are formed from a single sheet.

In addition, cutting action for forming the edges 1LE is not carried out on the inner body 3, thereby eliminating the risk of the inner body 3 being damaged by this cutting action. Thus, productivity of the diaper 1 can be maintained at a high level.

In addition, in the first embodiment according to the present invention, the connecting sheet 6 is not attached to the front piece web W4 and the back sheet web W5 in the form of a web, but rather is attached after having cut to an amount equivalent to a single finished product. As a result, costs incurred for the connecting sheet 6 can be reduced considerably. This is because, if the connecting sheet 6 is attached in the form of a web, the majority of the connecting sheet 6 ends up being removed by the cutting action.

Moreover, since the connecting sheet is attached discontinuously, the end portions 6SE can be positioned farther to the inside in the transverse direction LT than the side portions 4LS of the front piece 4 and the side portions 5LS of the back piece 5 that define the leg holes 1L. As a result, the connecting sheet 6 is not present around the side portions 4LS and 5LS, thereby improving comfort when worn.

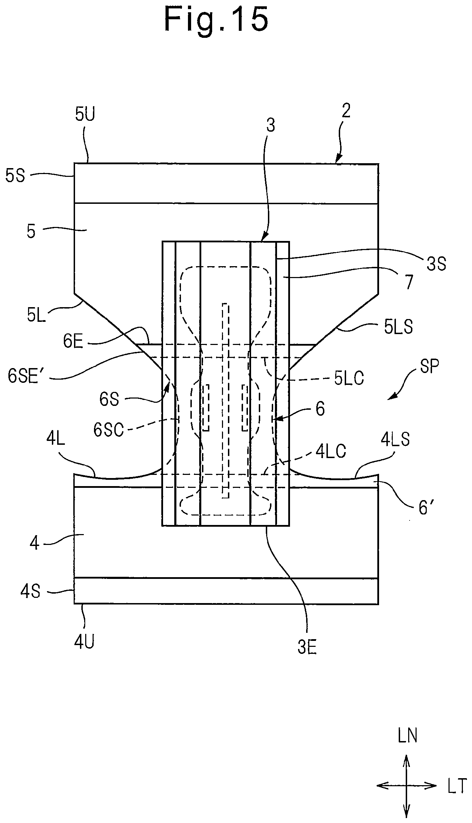

Namely, FIG 15 shows the case of the connecting sheet web W6 being attached to the front piece web W4 and the back piece web W5, and in this case, end portions 6SE' on the side of the back piece 5 coincide with the side portions 5LS, and the connecting sheet 6 is therefore present around the side portions 5LS. In addition, portions 6' of the connecting sheet 6 that overlap the front piece 4 extend to the side edges 4S of the front piece 4 along the side portions 4LS, and the connecting sheet 6 is therefore present around the side portions 4LS. As a result, the areas around the side portions 4LS and 5LS become comparatively hard, thereby resulting in the risk of imparting a sense of discomfort to the legs of a wearer.

In contrast, in the first embodiment according to the present invention, the end portions 6SE are positioned farther to the inside in the transverse direction LT than the side portions 4LS and 5LS. Thus, the sense of comfort is improved. Moreover, since the surface area of the connecting sheet 6 can be decreased, material costs can be reduced. Note that the end portions 6SE are not necessarily required to extend in the transverse direction LT, but rather may also have a curved shape. In addition, only one of the end portions 6SE on the side of the front piece 4 and the end portions 6SE on the side of the back piece 5 may be positioned farther to the inside in the transverse direction LT than the side portions 4LS and 5LS.

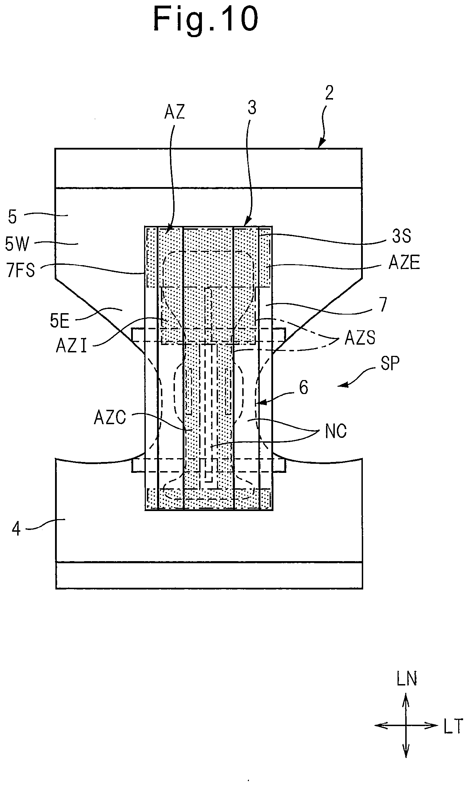

FIG. 10 shows an adhesive application pattern in step ST16.

As shown in FIG. 10, the width of an area AZ where an adhesive is applied varies along the longitudinal direction LN. Namely, the adhesive application area AZ has end areas AZE located on both ends in the longitudinal direction LN, a central area AZC located in the center in the longitudinal direction LN, and an intermediate area AZI located between the end area AZE and the central area AZC on the side of the back piece 5. Both end areas AZE and the intermediate area AZI do not overlap with the connecting piece 6, but rather the central area AZC overlaps with the connecting sheet 6.

The widths of both end areas AZE substantially coincide with the entire width of the inner body 3 and the leakage preventing members 7, and constitutes the largest width. This is to prevent the inner body 3 and the leakage preventing members 7 from separating from the front piece 4 and the back piece 5 and eliminate a sense of discomfort on the part of a wearer.

The width of the central area AZC is narrower than the width of the inner body 3 and constitutes the smallest width. In the first embodiment according to the present invention, the width of the central area AZC is about 90 mm.

The width of the intermediate area AZI is intermediate to the width of both end areas AZE and the central area AZC.

In this case, since adhesive is not applied to both sides in the transverse direction LT of the central area AZC and the intermediate area AZI, non-anchored areas NC are formed where the inner body 3 and the leakage preventing members 7 are not anchored to the outer body 2. The width of the non-anchored area NC around the central area AZC is larger than the width of the non-anchored area NC around the intermediate area AZI. Moreover, a non-anchored area NC is also formed in the center in the transverse direction of the central area AZC.

In this manner, a portion around the outer body 2, namely both edges 6S of the connecting sheet 6, and the inner body 3 are not mutually anchored between the front piece 4 and the back piece 5. Thus, the inner body 3 easily deforms corresponding to the physique of the wearer thereby enhancing adherence of the inner body 3.

The leakage preventing members 7 rise up by using as starting points edges AZS located on both sides in the transverse direction of the central area AZC and the intermediate area AZI. Thus, as a result of increasing the size of the noun-anchored area NC around the central area AZC, the amount of the rise of the leakage preventing members 7 can be increased. In addition, adherence of the inner body 3 to the wearer can be enhanced.

In contrast, the non-anchored area NC around the intermediate area AZI is reduced in size, thereby suppressing rising of the leakage preventing members 7. As a result, excessive rising of the leakage preventing members 7 that causes them to cover the absorptive surface of the inner body 3 is prevented. In addition, if the intermediate area AZI is provided on the side of the back piece 5, namely the gluteal region of the wearer, the leakage preventing members 7 can be prevented from riding up into the seat of the gluteal region, thereby enhancing the sense of comfort.

Note that, in step ST16 (FIG. 5), the previously described pattern formation can be facilitated by applying adhesive in the machine direction MD of the inner body web W3 and the leakage preventing member webs W7.

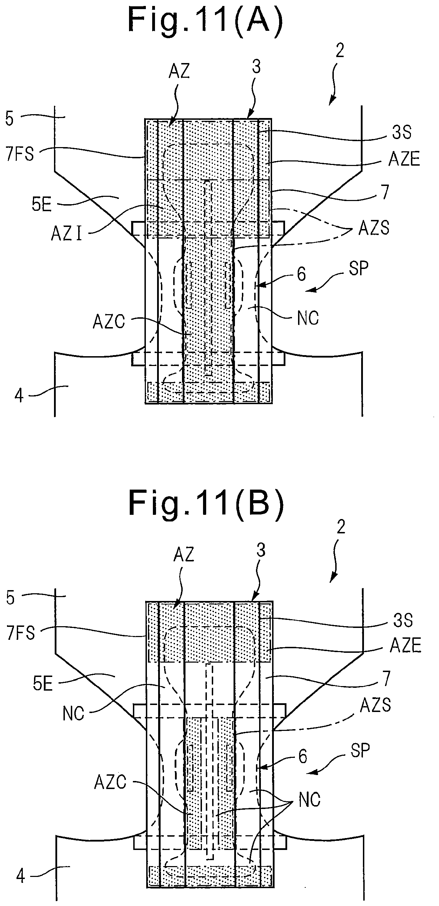

Note that, as shown in FIG. 11(A), the width of the intermediate area AZI may be made to substantially coincide with the entire width of the inner body 3 and the leakage preventing members 7. In this case as well, excessive twisting of the leakage preventing members 7 can be prevented. Note that the non-anchored area NC in the center in the longitudinal direction LN is omitted in this example.

Alternatively, as shown in FIG. 11(B), non-anchored areas NC may also be provided adjacent to both end areas AZE. This being the case, wrinkling and twisting of the inner body 3 are suppressed. As a result, the sense of comfort is enhanced and stable absorptive performance of the inner body 3 is maintained.

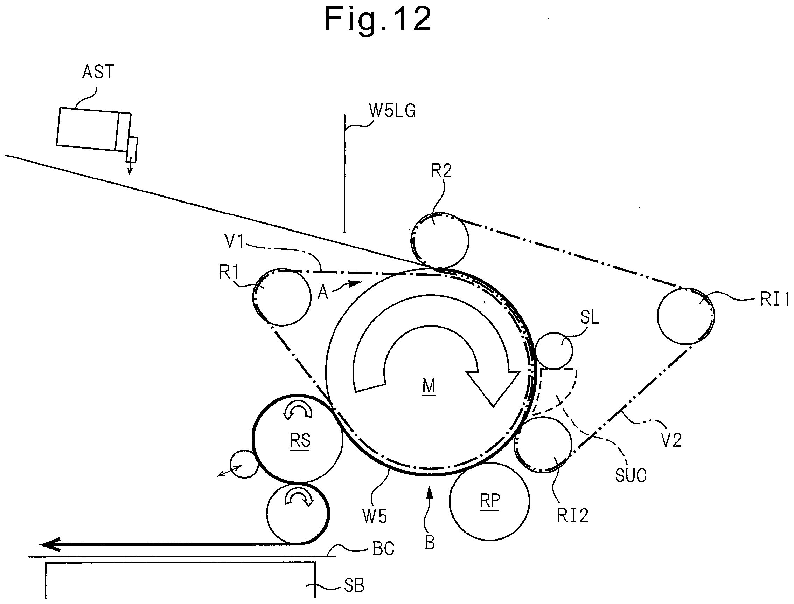

FIG. 12 shows a second embodiment according to the present disclosure.

In this second embodiment according to the present disclosure, the elastic members 5LG are discontinuously provided in the back piece 5. In addition, the elastic members 5LG are formed from an elastic sheet.

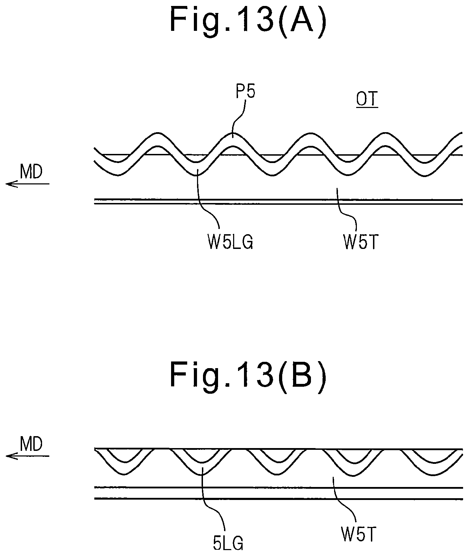

Namely, in the second embodiment as shown in FIG. 12, the top sheet web W5T is transported towards the main drum M. Prior to reaching the main drum M, the stretched continuous elastic body W5LG is supplied and attached to the top sheet web W5T while oscillating in the cross-machine direction CD. In this case, as shown in FIG. 13(A), the continuous elastic body W5LG is supplied so as to be alternately positioned on the top sheet web W5T and to the outside OT the top sheet web W5T.

Prior to reaching the main drum M, HMA is applied to the top sheet web 5WT from the HMA applicator AST so as to coincide with the arrangement pattern of the continuous elastic body W5LG. As a result, the portions of the continuous elastic body W5LG positioned on the top sheet web W5T are attached to the top sheet web W5T. Note that the rollers R1 and R2 are not mutually adjacent.

The endless belts V1 and V2 are fed to the main drum M so as to not overlap with the top sheet web W5T, and these endless belts V1 and V2 hold the portions P5 of the continuous elastic body W5LG positioned outside the top sheet web W5T. Note that, in this second embodiment according to the present invention, the endless belt V1 is formed by a single flat belt, while the endless belt V2 is formed by two wire belts.

Next, the portions P5 are cut by the slicer SL and removed. As shown in FIG. 13(B), the elastic members 5LG are intermittently or discontinuously provided in the top sheet web W5T. Next, the continuous elastic body W5LG is pressed onto the top sheet web W5T by the press roller RP. In this manner, in the second embodiment according to the present invention, the portions P5 are cut and removed without sandwiching the continuous elastic body W5LG between the top sheet web W5T and the back sheet web W5B.

Next, the continuous elastic body W5WG is supplied to the top sheet web W5T to form the folded portion 5F (see FIG. 3 etc.) and resulting in the formation of the back piece web W5. Next, the back piece web W5 is transported alongside the front piece web W4 in the spacing area SP in the cross-machine direction CD. Next, the outer body web S2 is formed, cutting action is carried out to form the edges 1LE that define the leg holes 1L, and the inner body 3 is attached to the outer body web W2.

In addition, in the second embodiment according to the present invention, the press roller RP is provided downstream from the slicer SL. This being the case, the length of the endless belts V1 and V2 can be shortened. As a result, deflection of the endless belts V1 and V2 during long-term operation is suppressed.

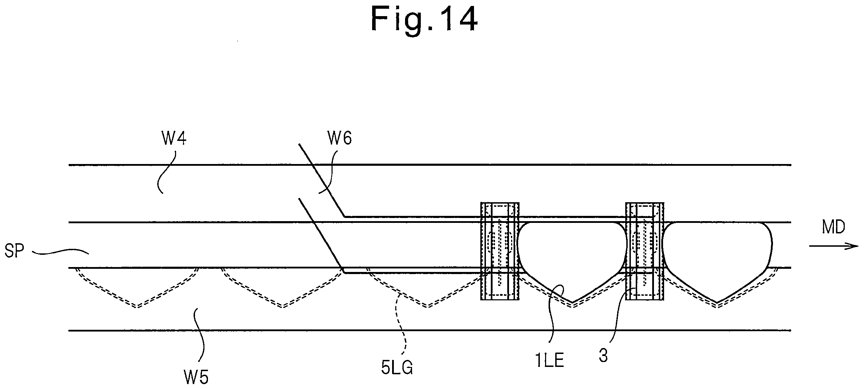

FIG. 14 shows a third embodiment according to the present disclosure.

In this third embodiment, although the elastic member 5LG is provided in the back piece 5, the elastic member 4LG is not provided in the front piece 4. Alternatively, the elastic member 4LG can be provided in the front piece 4, and the elastic member 5LG is not provided in the back piece 5.

In addition, the connecting sheet 6 is attached to the front piece web W4 and the back piece web W5 in the form of the connecting sheet web W6. Next, the inner body 3 is attached by overlapping with the connecting sheet web W6. Next, cutting action is carried out for forming the edges 1LE that define the leg holes 1L, thereby resulting in the formation of the connecting sheet 6 having narrowed portions.

Note that, in each of the previously described embodiments, the outer body 2 includes the connecting sheet 6. However, the outer body 2 can also be formed without including the connecting sheet 6. In the case of such an outer body 2, the inner body 3 is respectively attached to the inner surface or outer surface of the front piece 4 and the back piece 5 so as to be positioned in the crotch area 1C. In this case, the inner body 3 is partially or completely exposed to the outside.

Each of the previously described embodiments can also be mutually combined.

### Reference Signs List

- 1: Diaper
- 1C: Crotch area
- 1F: Side areas
- 1L: Leg holes
- 2: Outer body
- 3: Inner body
- 4: Front piece
- 5: Back piece
- 6: Connecting sheet
- 7: Leakage preventing members
- SP: Spacing area

## Claims

1. A manufacturing method of an underpants-type absorbent article, which is provided with
an outer body provided with a front piece and back piece mutually joined at side areas, the outer body having elastic members provided discontinuously along portions of the lower edges of the front piece and back piece that define leg holes, in which the outer body is provided with a connecting sheet which extends in the front-to-rear direction in a crotch area and mutually connects the front piece and the back piece, and
an inner body containing an absorptive body and which is anchored to the outer body so as to be positioned in a crotch area, comprising, in turn:
a step in which a continuous body of a sheet that forms the front piece or the back piece in the form of a sheet web is transported in a machine direction, wherein continuous bodies of sheets that respectively form the front piece and the back piece in the form of two sheet webs are respectively transported along the machine direction while maintaining a spacing area in the cross-machined direction,
a step in which a continuous body of an elastic member in the form of a continuous elastic body is supplied to the sheet web while oscillating in a cross-machine direction perpendicular to the machine direction so that the continuous elastic body is alternately positioned on the sheet web and outside the sheet web, and the portions of the continuous elastic body positioned on the sheet web are attached to the sheet web,
a step in which portions of the continuous elastic body positioned outside the sheet web are held by a holder,
a cutting step in which the portions of the continuous elastic body outside the sheet web are cut and removed
a step in which the two sheet webs are transferred onto a suction transfer roller, and then the connecting sheet is attached to the two sheet webs, positioned on the suction transfer roller, so as to straddle the spacing area and form a continuous body of the outer body in the form of an outer body web, wherein the connecting sheet overlaps a part of the elastic members,
an edge forming step in which the outer body web is cut to form edges that define leg openings and to position at least a portion of both side edges of the connecting sheet farther to the inside in the transverse direction than both side edges of the inner body, and
an inner body attachment step in which the inner body is attached to the outer body web after the edge forming step.

2. The manufacturing method according to claim 1, wherein the continuous elastic body is supplied so as to be alternately positioned on a single sheet web and within the spacing area.

3. The manufacturing method according to claim 1 or claim 2, wherein, after the continuous elastic body is attached on the sheet web, an additional sheet web is overlapped with the sheet web and the continuous elastic body is sandwiched between these sheet webs.

4. The manufacturing method according to any of claims 1 to 3, wherein the holder is provided with a pair of holding members that move with the sheet web, and the portions of the continuous elastic body outside the sheet web are cut while held between the holding members.

5. The manufacturing method according to claim 4, wherein the pair of holding members are formed by a wire belt and a flat belt having a concave groove capable of receiving the wire belt, and the portions of the continuous elastic body outside the sheet web are held as a result of the wire belt being accommodated in the concave groove of the flat belt together with those portions.

6. The manufacturing method according to claim 1, wherein the connecting sheet is attached to the sheet webs at intervals in the machine direction.

## Patentansprüche

1. Herstellungsverfahren eines unterhosenartigen, absorbierenden Artikels, welcher versehen ist mit
einem äußeren Körper, der mit einem Vorderteil und einem Hinterteil versehen ist, die in Seitenbereichen miteinander verbunden sind, wobei der äußere Körper elastische Elemente aufweist, die diskontinuierlich entlang von Abschnitten der unteren, Beinöffnungen definierenden Kanten des Vorderteils und Hinterteils vorgesehen sind, in welchen der äußere Körper mit einer Verbindungslage versehen ist, die sich in der von vorn nach hinten verlaufenden Richtung in einen Schrittbereich erstreckt und Vorderteil und Hinterteil miteinander verbindet, und
einem inneren Körper, der einen absorbierenden Körper umfasst und der an dem äußeren Körper verankert ist, sodass er in einem Schrittbereich angeordnet ist, wiederum enthaltend:
einen Schritt, bei dem ein kontinuierlicher Körper einer das Vorderteil oder das Hinterteil bildenden Lage in der Form einer Lagenbahn in eine Maschinenrichtung transportiert wird, wobei kontinuierliche Körper von Lagen, die jeweils das Vorderteil und das Hinterteil bilden, in der Form von zwei Lagenbahnen jeweils entlang der Maschinenrichtung transportiert werden, während ein Abstandsbereich in die Maschinenquerrichtung beibehalten wird,
einen Schritt, bei dem ein kontinuierlicher Körper eines elastischen Elements in der Form eines kontinuierlichen elastischen Körpers der Lagenbahn zugeführt wird, während er in eine zu der Maschinenrichtung senkrechte Maschinenquerrichtung oszilliert, sodass der kontinuierliche elastische Körper abwechselnd auf der Lagenbahn und außerhalb der Lagenbahn angeordnet ist und die Abschnitte des kontinuierlichen elastischen Körpers, die auf der Lagenbahn angeordnet sind, an der Lagenbahn angebracht sind,
einen Schritt, bei dem Abschnitte des kontinuierlichen elastischen Körpers, die außerhalb der Lagenbahn angeordnet sind, von einem Halter gehalten werden,
einen Schneideschritt, bei dem die Abschnitte des kontinuierlichen elastischen Körpers außerhalb der Lagenbahn geschnitten und entfernt werden,
einen Schritt, bei dem die beiden Lagenbahnen auf eine Saug-Transferwalze übertragen werden und dann die Verbindungslage an die beiden Lagenbahnen, die auf der Saug-Transferwalze angeordnet sind, befestigt wird, sodass sie den Abstandsbereich überspannt und einen kontinuierlichen Körper des äußeren Körpers in der Form einer äußeren Körper-Bahn bildet, wobei die Verbindungslage einen Teil der elastischen Elemente überlappt,
einen Kantenbildungsschritt, bei dem die äußere Körper-Bahn geschnitten wird, um Kanten zu bilden, die Beinöffnungen bestimmen und um zumindest einen Abschnitt von beiden Seitenkanten der Verbindungslage weiter in der Innenseite in die Querrichtung anzuordnen, als beide Seitenkanten des inneren Körpers, und
einen inneren Körper Befestigungsschritt, bei dem der innere Körper an der äußeren Körper-Bahn nach dem Kantenbildungsschritt befestigt wird.

2. Herstellungsverfahren nach Anspruch 1, wobei der kontinuierliche elastische so Körper zugeführt ist, dass er abwechselnd auf einer einzelnen Lagenbahn und in dem Abstandsbereich angeordnet ist.

3. Herstellungsverfahren nach Anspruch 1 oder 2, wobei, nach dem der kontinuierliche elastische Körper auf der Lagenbahn befestigt ist, eine zusätzliche Lagenbahn mit der Lagenbahn überlappt wird und der kontinuierliche elastische Körper zwischen diesen Lagenbahnen sandwichartig angeordnet wird.

4. Herstellungsverfahren nach einem der Ansprüche 1 bis 3, wobei der Halter mit einem Paar von Halteelementen vorgesehen ist, die sich mit der Lagenbahn bewegen, und die Abschnitte des kontinuierlichen elastischen Körpers außerhalb der Lagenbahn geschnitten werden, während sie zwischen den Halteelementen gehalten werden.

5. Herstellungsverfahren nach Anspruch 4, wobei das Paar von Halteelementen durch ein Drahtband und ein Flachband mit einer konkaven Nut, die fähig ist das Drahtband aufzunehmen, gebildet ist und die Abschnitte des kontinuierlichen elastischen Körpers außerhalb der Lagenbahn als ein Ergebnis der Aufnahme der Drahtbahn zusammen mit diesen Abschnitten in der konkaven Nut des Flachbands gehalten sind.

6. Herstellungsverfahren nach Anspruch 1, wobei die Verbindungslage an den Lagenbahnen in Intervallen in die Maschinenrichtung befestigt ist.

## Revendications

1. Procédé de fabrication d'un article absorbant de type culotte, qui est doté
d'un corps extérieur doté d'un morceau avant et d'un morceau arrière assemblés mutuellement au niveau de zones latérales, le corps extérieur ayant des organes élastiques prévus en discontinu le long de portions des bords inférieurs du morceau avant et du morceau arrière qui définissent des trous pour les jambes, dans lequel le corps extérieur est doté d'une feuille de liaison qui s'étend dans la direction d'avant en arrière dans une zone d'entrejambe et relie mutuellement le morceau avant et le morceau arrière, et
d'un corps intérieur contenant un corps absorbant et qui est ancré au corps extérieur de façon à être positionné dans une zone d'entrejambe, comprenant, tour à tour :
une étape dans laquelle un corps continu d'une feuille qui forme le morceau avant ou le morceau arrière sous forme d'une bande de feuille est transporté dans un sens machine, dans lequel des corps continus de feuilles qui forment respectivement le morceau avant et le morceau arrière sous la forme de deux bandes de feuille sont transportés respectivement le long du sens machine tout en maintenant une zone d'espacement dans le sens travers, une étape dans laquelle un corps continu d'un organe élastique sous forme d'un corps élastique continu est fourni à la bande de feuille tout en oscillant dans un sens travers perpendiculaire au sens machine de sorte que le corps élastique continu soit positionné en alternance sur la bande de feuille et à l'extérieur de la bande de feuille, et les portions du corps élastique continu positionné sur la bande de feuille sont fixées à la bande de feuille,
une étape dans laquelle des portions du corps élastique continu positionné à l'extérieur de la bande de feuille sont retenues par un dispositif de retenue,
une étape de coupe dans laquelle les portions du corps élastique continu à l'extérieur de la bande de feuille sont coupées et retirées,
une étape dans laquelle les deux bandes de feuille sont transférées sur un rouleau de transfert aspirant, puis la feuille de liaison est fixée aux deux bandes de feuille, positionnée sur le rouleau de transfert aspirant, de façon à enjamber la zone d'espacement et former un corps continu du corps extérieur sous la forme d'une bande de corps extérieure, dans lequel la feuille de liaison chevauche une partie des organes élastiques,
une étape de formation de bord dans laquelle la bande de corps extérieur est coupée pour former des bords qui définissent des ouvertures pour les jambes et pour positionneur au moins une portion des deux bords latéraux de la feuille de liaison plus loin vers l'intérieur dans le sens machine que les deux bords latéraux du corps intérieur, et
une étape de fixation de corps intérieur dans laquelle le corps intérieur est fixé à la bande de corps extérieur après l'étape de formation de bord.

2. Procédé de fabrication selon la revendication 1, dans lequel le corps élastique continu est fourni de façon à être positionné en alternance sur une seule bande de feuille et au sein de la zone d'espacement.

3. Procédé de fabrication selon la revendication 1 ou la revendication 2, dans lequel, après que le corps élastique continu est fixé sur la bande de feuille, une bande de feuille supplémentaire est chevauchée avec la bande de feuille et le corps élastique continu est enserré entre ces deux bandes de feuille,

4. Procédé de fabrication selon l'une quelconque des revendications 1 à 3, dans lequel le dispositif de retenue est doté d'une paire d'organes de retenue qui se déplacent avec la bande de feuille, et les portions du corps élastique continu à l'extérieur de la bande de feuille sont coupées tout en étant retenues entre les organes de retenue.

5. Procédé de fabrication selon la revendication 4, dans lequel la paire d'organes de retenue est formée par une courroie métallique et une courroie plate ayant une gorge concave capable de recevoir la courroie métallique, et les portions du corps élastique continu à l'extérieur de la bande de feuille sont retenues en raison de la courroie métallique qui est logée dans la gorge concave de la courroie plate conjointement à ces portions.

6. Procédé de fabrication selon la revendication 1, dans lequel la feuille de liaison est fixée aux bandes de feuille à des intervalles dans le sens machine.
